# EUROPEAN PATENT SPECIFICATION

(11) **EP 0 572 271 B1**
(45) Date of publication and mention of the grant of the patent: **22.01.1997**
(21) Application number: 93304176.6
(22) Date of filing: 28.05.1993
(51) Int. Cl.: A01N 37/36, A61K 7/00

(54) **Acyl lactylates as antimicrobial actives**
Acyl Lactylate als antimikrobielle Mittel
Lactylates d'acyle comme agents antimicrobes

(30) Priority: 29.05.1992 GB 9211428
(43) Date of publication of application: 01.12.1993
(73) Proprietor: UNILEVER PLC, London EC4P 4BQ (GB); UNILEVER N.V., 3013 AL Rotterdam (NL)
(72) Inventor: Carter, Peter, Burton, Cheshire L64 5TL (GB); Morris, Christine, Upton by Chester CH2 1NB (GB); Hagan, Desmond Bernard, South Wirral, Cheshire L66 4TE (GB)
(74) Representative: Mole, Peter Geoffrey

(56) References cited:
- GB-A- 1 115 480

## Description

### Field of Invention

The invention relates to the use of specific acyl lactylates as antimicrobial actives, that is to inhibit or prevent the growth of microorganisms or to kill microorganisms present. In particular, the invention is concerned with the use of such acyl lactylates to preserve compositions which would otherwise be subject to or at risk of microbial spoilage.

### Background to the Invention

It is well known that the presence and the growth and proliferation of many bacteria, moulds, mildews and yeasts is undesired in many locations because of deterioration of the materials concerned, toxicological hazards, disease causing factors and similar considerations. For instance foods and feedstuffs may be subject to deterioration or spoilage because of growth of such microorganisms. Furthermore, certain skin diseases can be caused or initiated by the presence of specific types of fungi.

Compositions that deteriorate or spoil as a result of the presence and the growth of microorganisms can have a very short shelf life, that is the period during which they can usefully be employed, unless they are refrigerated, frozen or sterilised within a sealed container. For compositions which are intended for occasional or regular use by the consumer, for example as beauty products or cleansing products for the skin, hair or teeth, it is inconvenient to maintain them under refrigerated conditions, and normally impossible to prevent contamination by adventitious microorganisms, particularly yeast, moulds and bacteria, of an otherwise sterile product, once the packaging that maintains sterility is opened.

In order to prevent microbial spoilage of such products, it has hitherto been necessary to incorporate a preservative chemical, such as 2-bromo-2-nitropropane-1,3-diol (Bronopol) or methyl paraben, the level at which such preservatives are used being determined by the temperature at which the product is normally stored prior to each use and dependent also on the intrinsic properties of the composition itself in the provision of conditions that support the proliferation of microorganisms. In other words, high levels of preservative are necessary where the product is to be stored in a warm place or in tropical or sub-tropical conditions, or where the composition provides an excellent growth medium for adventitious microorganisms.

As research into the merits or demerits of using conventional preservative chemicals progresses, there is an increasing realisation that some of these chemicals can themselves deteriorate on storage, so that their preservative benefits diminish or are lost, or they can decompose in the product with the formation of bi-products that can be harmful to health. As an example, 2-bromo-2-nitropropane-1,3-diol has been shown under certain conditions to give rise to N-nitrosamines species which are alleged to be carcinogenic. Conventional preservatives can also be toxic in their own right unless used at very low levels at which their ability to prevent microbial spoilage is hardly adequate.

Another group of antimicrobial active compounds are the acyl lactylates as disclosed in the US patent 3,275,503 (C J Patterson Co.). It is described in this patent that acyl lactylates having an acyl radical derived from a C₈-C₁₂ fatty acid are capable of inhibiting the growth of specific types of microorganisms. The most effective compound appears to be a C₁₀ acyl lactylate, namely calcium capryl-2 lactylate. Although, this compound is very effective against several types of bacteria its efficacy to inhibit the growth of moulds, like aspergillus niger, leaves much to be desired.

It has now been discovered that a narrow range of short chain acyl lactylates is able to overcome the drawbacks attributed to conventional antimicrobial active compounds. Surprisingly, these specific acyl lactylates are not only very effective against proliferation of bacteria but also inhibit the growth of moulds in an unexpected degree.

We have also discovered that the preservative properties of these acyl lactylates acids in terms of their ability to inhibit the proliferation of microorganisms, can be further enhanced to an unexpected extent by employing them together with sub-optimal concentrations of conventional chemical preservatives. Thus, conventional chemical preservatives, whose normal effective level of use has given rise to safety problems, can now be employed at lower than usual, and hence safer, levels in the presence of one or more of these acyl lactylates.

### Definition of the Invention

Accordingly, the invention provides for the use of an acyl lactylate of the following structure (I) 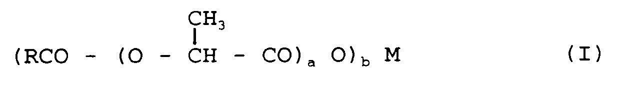 where RCO represents a C₄-C₇ acyl radical; M represents H or a counterion chosen from alkali metal, alkaline earth metal, zinc, silver, ammonium or substituted ammonium having one or more C₁-C₃ alkyl or hydroxy alkyl group(s); a is an integer of from 1 to 3; b is 1 or 2; or a mixture of such acyl lactylates; in the preparation of an antimicrobially active composition.

Compounds in which a is 1 are termed monolactylates.
Compounds in which a is 2 are termed dilactylates.

### Disclosure of the Invention

The invention is accordingly concerned with the use of acyl lactylates having the structure (1) as defined above in killing undesired microorganisms or inhibiting or preventing their growth. Notably, these microorganisms may be bacteria, moulds and yeasts.

Preferred acyl lactylates of the structure (1) are those where RCO represents a C₆ acyl radical. Particularly preferred examples are:
hexanoyl mono lactylic acid
sodium hexanoyl mono lactylate
calcium di-(hexanoyl mono lactylate)

M in structure (1) is preferably H or a counterion chosen from sodium, potassium or calcium.

The lactylates may take the form of mixtures of compounds with different numbers of lactylate residues, such as a mixture of meno and dilactylates.

The acyl lactylates employed in the use according to the invention have in particular been demonstrated to be effective against
Pseudomonas cepacia as bacteria;
a mixture of Aspergillus niger and Penicillium notatum as mould.

For treatment and prevention of diseases, for instance of the skin, which are caused by microorganisms, the acyl lactylates can be applied topically to the areas affected in the form of powders, ointments, solutions, gels or any other cosmetically acceptable type of composition, preferably the topically appliable compositions as defined below.

If the acyl lactylates are to be used as inhibitors or preventers of microbial spoilage of otherwise perishable products, they can be mixed with the products to be protected or such products may be impregnated or coated with the acyl lactylates. In any case, it is necessary that the acyl lactylates come into close contact with the area or material to be protected against growth of undesired microorganisms.

The acyl lactylates according to the invention are advantageous for their preservative effect, preferably when incorporated in compositions which are intended to be topically applied to the human body, especially the skin or hair. Their inclusion in such cosmetic composition counteracts effectively the growth of undesired microorganisms. As is shown in more detail in the examples the acyl lactylates are not only very effective against bacteria but also against moulds. This is surprising as conventional preservatives tend to show a high activity only against one of these two types of microorganisms.

### Compositions

The preferred compositions which are preserved by incorporation of the acyl lactylate employed in the use according to the invention normally comprise a cosmetically acceptable vehicle.

Vehicles other than water can include liquid or solid emollients, solvents, humectants, thickeners and powders. Examples of each of these types of vehicle which can be used singularly or as mixtures of one or more vehicles, are as follows:

Emollients, such as stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, di-n-butyl sebacate, isoporpyl myristate, isopropyl palmitate, isopropyl stearate, butyl stearate, polyethylene glycol, triethylene glycol, lanolin, cocoa butter, corn oil, cotton seed oil, olive oil, palm kernel oil, rapeseed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate;

Propellants, such a propane, butane, isobutane, dimethyl ether, carbon dioxide, nitrous oxide;

Solvents, such as ethyl alcohol, methylene chloride, isopropanol, acetone, ethylene glycol monoethyl ether, diethylene glucol monobutyl ether, diethylene glycol monoethyl ether, dimethyl sulphoxide, dimethyl formamide, tetrahydrofuran;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose, ethylene glycol monostearate.

The cosmetically acceptable vehicle will usually form from 10 to 99.9%, preferably from 50 to 99% by weight of the composition, and can, in the absence of other cosmetic adjuncts, form the balance of the composition.

### pH

The composition which can be protected against microbial spoilage according to the invention should preferably have a pH value not exceeding pH 7. Ideally, the pH value of the composition is from pH 4 to pH 7.

Adjustment of pH can be achieved by addition of a pH adjustant as conventionally used in the cosmetics art.

### Conventional Chemical Preservatives

The composition for use according to the invention can optionally comprise one or more conventional chemical preservatives, preferably at a concentration that is lower than their optional level of use. Accordingly, these conventional chemical preservatives that may, when used alone, give rise to safety problem, when employed in sufficient quantity to prevent microbial spoilage, can in the presence of one or more acyl lactylates, as herein defined, be effective as preservatives as acyl lactylates are employed together with one or more conventional chemical preservatives, then the effectiveness of such mixtures in preventing microbial spoilage can be very high.

Examples of chemical preservatives that can be employed in this way include:
formaldehyde
2-bromo-2-nitropropane-1,3-diol: (eg. Bronopol or Myacide BT)
methyl paraben: (eg. Nipagin M)
propyl paraben: (eg. Nipasol M)
butyl paraben: (eg. Nipabutyl)
imidazolidinyl urea: (eg. Germall 115)
diazolidinyl urea: (eg. Germall II)
sorbic acid/potassium sorbate
benzoic acid/sodium benzoate
salicylic acid/sodium salicylate
phenoxyethanol
phenoxyethanol (80%) + dibromodicyanobutane (20%): (eg. Euxyl K400)
dimethylol dimethyl hydantoin: (eg. Glydant)
Chloroacetamine
dehydroacetic acid
**glutaraldehyde**
methyl chloro isothiazolinone
phenyl ethyl acetate
1-(3-chloroallyl)-3,5,7-triaza-1-azonia adamantane chloride: (eg. Quaternium 15)
sodium hydroxymethylglycinate
benzyl alcohol

Chemical preservatives can be employed individually or in mixtures, together with the acyl lactylates(s).

The acyl lactylates for the use according to the invention can be incorporated in the compositions to be protected in an amount of from 0.01 to 10, preferably 0.1 to 3% by weight, based on the weight of the composition.

The amount of chemical preservative when employed will normally be at least 10% less than that normally necessary, in the absence of the acyl lactylate to inhibit microbial proliferation.

### COSMETIC ADJUNCTS

The composition for the use in accordance with the invention optionally can comprise cosmetic adjuncts. Examples are as follows:
Organic sunscreen materials in an amount of from 0.1 to 20%, preferably from 1 to 10% by weight;
Inorganic sunscreens, such as ultra fine titanium dioxide;
Emulsion ingredients, these are present if the composition for the use according to the invention is in the form of an emulsion in which case an oil or oily material will normally be present, together with an emulsifier to provide either a water-in-oil emulsion or an oil-in-water emulsion. Examples of suitable oils and oily materials include mineral oil, vegetable oils, silicone oils, both volatile and non-volatile, such as polydimethyl siloxanes. The oil or oily material when present for the purposes for forming an emulsion, will normally form up to 90%, preferably from 10 to 80% by volume of the composition. The amount of the emulsifier or mixtures thereof will normally be from 1 to 50%, preferably from 2 to 20% and most preferably from 2 to 10% by weight of the composition. The composition can also comprise of water, usually up to 80%, preferably from 5 to 80% by volume.
Surfactants, such as anionic, non-anionic or amphoteric surfactants or mixtures thereof, particularly when the composition is intended for shampooing the hair or for use when bathing or in the shower. When present the surfactants form from 2 to 40 % by weight and preferably from 5 to 30 % by weight.
Other Cosmetic Adjuncts, which include for instance antioxidants, such as butyl hydroxy toluene; humectants such as glycerol, sorbitol; buffers such as lactic acid or sodium hydroxide; waxes such as beeswax, paraffin wax; plant extracts, such as aloa vera; thickeners; colourants; and perfumes.

The acyl lactylates, as herein defined or mixtures thereof can be used to preserve a wide variety of compositions intended for topical application to the human body. These compositions can, for example, include skin creams, lotions, milks and powders, skin cleansing products, hair treatment products, such as shampoos, conditioners, styling aids and dental products, such as toothpastes and mouthwashes.

Furthermore, the compositions as defined above can also serve as a vehicle to distribute the acyl lactylates to areas such as the human skin, which are effected by undesired microorganisms, in particular fungi.

### Challenge Test Procedure for evaluating antimicrobial activity of preservatives in compositions for the use of the invention

A test was devised to assess the ability of selected acyl lactylates to preserve, against microbial spoilage, compositions according to the invention and, by way of comparison, others containing conventional chemical preservative substances.

This test involved incubating under standard conditions test compositions which have been inoculated with a selected bacterium, yeast or a mixture of moulds. Each test composition was repeatedly inoculated at regular intervals ("challenged"), until the surviving microorganisms from the inoculation showed an increase above a standard level, at which point, the preservative system under test was judged to have failed. Accordingly, the higher the number of "challenges", the better is the preservative substance in preventing or at least delaying microbial spoilage.

### Materials

A series of compositions containing acyl lactylates chosen from those in accordance with the invention or conventional chemical preservatives was prepared.

The microorganisms selected for this test were:
i) Pseudomonas cepacia - a gram-negative bacterium,
ii ) Aspergillus niger & Penicillium notatum, - a mixture of moulds.

The formulations used in the Challenge Test were creams as set out in the Results section below.

### Method

For the bacterium the method of testing was as follows:
i) Introduce aseptically 99ml of test composition into a 250ml flask and add 1ml of inoculum of one of the test organisms (see above), to provide a dilution of 106 cells/ml in the composition.
ii) mix and incubate at 28°C for 24 hours.
iii) remove 1ml of incubated composition from the flask and add to it as diluent 9ml of an aqueous solution containing 0.1% by weight peptone and 2% by weight Tween 80, and mix thoroughly.
iv) place 1ml of diluted sample into a petri dish and add warm, molten agar (40°C) and mix to distribute culture uniformly and allow to set.
v) incubate plates for 3 days at 28°C and then count colonies and note log reduction of count (due to the presence of the preservative in the original culture composition).

The inoculation of the organisms originally introduced into each composition is then repeated at 24 hour intervals, following removal of the first total viable count sample, the plating out and counting procedure being repeated on each occasion. The test is terminated when the samples show a contamination level greater than 10² counts per ml on 2 consecutive days. This point is regarded as the failure point for the composition under test.

The greater the number of inoculations of contaminant organisms before this level of contamination is established in the composition, the better the preservative system.

The above procedure applies to the inoculation of the bacterium. For testing with the mixed culture of moulds this procedure was varied slightly, as follows:

Two samples of each test composition (9.9ml and 9ml) were transferred to sterile flasks and 0.1ml or 1ml, (i.e. 1% or 10% by volume) of a mould spore suspension (approximately 10⁸ spores per ml) was introduced into the respective samples and thoroughly mixed. The inoculated samples were incubated at 28°C for up to 28 days with 1ml samples being aseptically removed for the total viable count as outlines above using Sabourand's dextrose agar after 1, 7, and 28 days.

A composition is classed as "low risk" if mould spores are eliminated in 1 to 2 days. "Medium risk" compositions take 7 to 14 days to eliminate the spores and "high risk" compositions still have surviving mould spores after 14 days incubation.

### Example 1

Six experiments were conducted to study the benefits of using the acyl lactylates, compared with conventional chemical preservatives, in preventing microbial spoilage of creams which were water-in-oil emulsions.

In each experiment sodium hydroxide or hydrochloric acid was used to adjust the pH to a value of 5.5.

The formulations used are set out in Table I overleaf:

**Table I**

| **Ingredient** | **I** | **II** | **III** | **IV** | **V** | **VI** |
|---|---|---|---|---|---|---|
| No preservative | - | - | - | - | - | - |
| Lactic acid | - | 1.0 | - | - | - | - |
| Hexanoyl lactylic acid | - | - | 1.0 | - | - | - |
| Octanoyl lactylic acid | - | - | - | 1.0 | - | - |
| Sodium capryl/Sodium lauryl | | | | | | |
| lactylate mixture (1:1) | - | - | - | - | 1.0 | - |
| Sodium stearoyl lactylate | - | - | - | - | - | 1.0 |
| Butane-1,3-diol | 10.0 | 10.0 | 10.0 | 10.0 | 10.0 | 10.0 |
| Sodium chloride | 2.0 | 2.0 | 2.0 | 2.0 | 2.0 | 0.20 |
| Silicone oil, DC344 | 8.2 | 8.2 | 8.2 | 8.2 | 8.2 | 8.2 |
| Silicone oil, DC3225C | 12.0 | 12.0 | 12.0 | 12.0 | 12.0 | 12.0 |
| TiO₂ Tiona AG | 0.2 | 0.2 | 0.2 | 0.2 | 0.2 | 0.2 |
| Petroleum jelly | 0.5 | 0.5 | 0.5 | 0.5 | 0.5 | 0.5 |
| Mineral oil | 1.5 | 1.5 | 1.5 | 1.5 | 1.5 | 1.5 |
| Perfume (Ayesha SN32667) | 0.15 | 0.15 | 0.15 | 0.15 | 0.15 | 0.15 |
| Water (demin.) | <---- | ----- | ---to | 100%- | ----- | ----> |
| pH | <---- | ----- | ----- | 5.5-- | ----- | ----> |

The results are shown in Table 2 below:

**Table 2**

| Formulation Code | Number of inoculations to failure Bacteria | Mould challenge 1% |
|---|---|---|
| I | 1 | ++++ |
| II | >23 | ++ |
| III (Invention) | >23 | - |
| IV | >23 | + |
| V | 1 | - |
| VI | 1 | ++ |
| **Key to Mould Challenge (after injection of spores)** - No mould growth 1, 7, 14 or 28 days + Mould growth 1 day after; no growth after 7, 14 or 28 days ++ Mould growth 1 and 7 days after; no growth after 14 or 28 days +++ Mould growth 1, 7 and 14 days after; no growth after 28 days ++++ Mould growth 1, 7, 14 and 28 days after injection of spores. | | |

These results indicate that Hexanoyl lactylic acid (formulation III) is superior in terms of antimicrobial activity against bacteria as well as moulds compared to conventional preservatives, like lactic acid (formulation II) and conventional acyl lactylates (formulations IV, V and VI). Although the conventional acyl lactylate used in formulation IV shows a very good activity against bacteria it fails to provide a similar inhibiting effect against moulds. In contrast to this, the acyl lactylate for the use according to the invention (formulation III) does not only show high efficacy against bacteria but also a very quick elimination of all moulds even if it is only used in a concentration of 1% by weight of the base composition. Such a high activity against moulds can only be achieved by using the conventional acyl lactylate mixture employed in formulation V, but with the disadvantage that the growth of bacteria is not at all inhibited.

### Example 2

Similar challenge test experiments were carried out using oil-in-water emulsions, the bacterian and the moulds. For all of the experiments in this example, the emulsions contained:

| | % wt |
|---|---|
| Xanthan gum (thickener) | 0.50 |
| Butane-1,3-diol (humectant) | 13.50 |
| Lanolin alcohols | 1.35 |
| Petroleum wax | 2.56 |
| Glyceryl monostearate | 3.84 |
| Cetyl alcohol [20EO] ethoxylate | 1.40 |
| Cetyl alcohol | 0.60 |
| Stearyl alcohol | 0.60 |
| Titanium dioxide | 0.15 |
| Silicone oil DC345 | 7.60 |
| Sodium hydroxide (20% AQ. Soln) | to give pH 5.5 |
| Demineralised water | balance to 100.0% |

To this base formulation there were added various materials for testing. The amount added was usually 1.0 wt%, but some conventional preservatives were used at lower levels, customary for such materials.

The materials and the results are set out in the following Table 3.

| Material added | No of inoculations to failure (Bacteria) | Mould challenge | |
|---|---|---|---|
| | | 1% | 10% |
| None (control) | 1 | ++++ | ++++ |
| Hexanoyl lactylate | >21 | + | + |
| Octanoyl lactylate | 20 | + | ++++ |
| Lauric acid | 1 | ++++ | ++++ |
| Monolaurin | 1 | ++++ | ++++ |
| Mixed preservatives | >20 | + | + |
| Farnesol (0.3 wt%) | 1 | ++++ | ++++ |

The mixed preservatives were
0.2 wt% methyl paraben
0.1 wt% propyl paraben
0.5 wt% butyl hydroxy toluene
0.01 wt% 2-bromo-2-nitropropane-1,3-diol (Bronopol)

Farnesol is an acyclic primary sesquiterpene alcohol marketed by Dragoco as a nature identical bacteriostat 0.3 wt% is the recommended dosage.

As can be seen from Table 3, hexanoyl lactylate outperfumed all the other materials tested, except for the mixed preservatives. This test did not determine whether or not the mixed preservatives gave better performance than hexanoyl lactylate.

### Example 3

Challenge test experiments were carried out using a shampoo formulation and the bacterium.

For all of these experiments, a shampoo formulations was used, containing:

| | % by weight |
|---|---|
| Sodium lauryl ether (2EO) sulphate | 11 |
| Lauryl dimethyl betaine | 2 |
| Coconut monoethanolamide | 1 |
| Sodium chloride | 1.3 |
| Citric acid | to give pH 6.0 |
| Water | balance to 100% |

Various materials were added to this shampoo formulation for testing against bacterial contamination, using the above challenge test procedure. The amount of each material added was 1% by weight. The results are set out in the following Table 4.

| Material added (1 wt%) | No of inoculations to failure (bacteria) |
|---|---|
| None | 1 |
| Hexanoyl lactylate | >21 |
| Octanoyl lactylate | 2 |
| Lauric acid | 1 |
| Monolaurin | 1 |

### Example 4

Challenge test experiments similar to Example 1 were carried out using oil-in-water creams containing:

| | % by weight |
|---|---|
| Silicone oil DC3225C | 12 |
| Silicone oil DC344 | 8.2 |
| Titanium dioxide | 0.2 |
| Petroleum jelly | 0.5 |
| Mineral oil | 1.5 |
| Water | balance to 100% |

Materials added to this base formulations, and the challenge test results, are shown in the following Table:

| | % by weight | | | | |
|---|---|---|---|---|---|
| Hexanoyl lactylate | -- | -- | -- | 1.0 | 0.5 |
| Octanoyl lactylate | -- | -- | 0.5 | -- | -- |
| Methyl paraben | -- | 0.2 | 0.1 | 0.1 | 0.2 |
| Propyl paraben | -- | 0.1 | 0.05 | 0.05 | 0.05 |
| No of inoculations to failure (bacteria) | -- | >21 | 2 | >21 | >21 |
| Mould challenge 1% | +++ | + | ++++ | -- | NC |
| Mould challenge 10% | ++++ | + | ++++ | + | NC |
| NC = not tested with mould | | | | | |

### Example 5

A sunscreen formulation was prepared containing:

| | % by weight |
|---|---|
| Butane-1,3-diol | 10 |
| Sodium chloride | 2 |
| Parsol MCX | 3 |
| Parsol 1789 | 1 |
| Silicone oil DC3225C | 12 |
| Silicone oil DC344 | 7 |
| Titanium dioxide | 0.2 |
| Petroleum jelly | 0.5 |
| Mineral oil | 1.5 |
| Hexanoyl lactylate | 1.0 |
| Water | balance to 100% |
| pH was adjusted to 5.5 with citric acid or triethanolamine as required. | |

When subjected to the challenge test with bacteria, the number of inoculations to failure exceeded 21.

This invention is further illustrated by the following additional examples of formulations of compositions preserved by the incorporation of the acyl lactylates for the use according to the invention.

### Example 6

| **Shampoo** | |
|---|---|
| | **% w/w** |
| Hexanoyl Lactylic Acid | 1.00 |
| Sodium Lauryl Ethoxy (2EO) Sulphate | 11.00 |
| Lauryl Dimethyl Betaine | 1.00 |
| Coconut Diethanolamide | 2.00 |
| Sodium Chloride | 0.50 |
| Sodium Hydroxide | to pH 6.3 |
| Demineralised Water | to 100.00 |

### Example 7

| **Skin-Lotion** | |
|---|---|
| | **% w/w** |
| Sodium Hexanoyl Monolactylate | 1.00 |
| Glycerol | 5.00 |
| Glycerol Monostearate | 1.00 |
| Mineral Oil | 3.00 |
| Carbopol 941 (Polyacrylate-Thickener) | 0.10 |
| Stearic Acid | 3.00 |
| Cetyl Alcohol | 1.00 |
| Sodium Hydroxide | to pH 7.0 |
| Demineralised Water | to 100.0 |

### Example 8

| **Skin Cream** (Water-in-Oil Emulsion) | |
|---|---|
| | **% w/w** |
| Calcium Hexanoyl Dilactylate | 1.50 |
| Butane-1,3-diol | 15.00 |
| Sodium Chloride | 3.00 |
| Silicone Oil | 15.00 |
| Petroleum Jelly | 0.50 |
| Mineral Oil | 1.50 |
| Demineralised water | to 100.00 |

### Example 9

| **Skin Cream** (Water-in-Oil Emulsion with reduced amounts of conventional preservatives) | |
|---|---|
| | **% w/w** |
| Sodium Hexanoyl Dilactylate | 1.00 |
| Glycerol | 10.00 |
| Sodium Chloride | 5.00 |
| Volatile Silicone Oil | 20.00 |
| Methyl Paraben (conventional Preservative) | 0.10 |
| Propyl Paraben (conventional Preservative) | 0.05 |
| Mineral Oil | 2.00 |
| Titanium Dioxide | 0.20 |
| Sodium Hydroxide | to pH 5.5 |
| Demineralised water | to 100.00 |

### Example 10

| **Skin Cream** (Oil-in-Water Emulsion) | |
|---|---|
| | **% w/w** |
| Silver Hexanoyl Lactylate | 1.25 |
| Xanthan Gum | 0.50 |
| Propane diol | 13.00 |
| Lanolin Alcohol | 1.50 |
| Glycerol Monostearate | 4.00 |
| Hexadecanol | 1.00 |
| Octadecanol | 1.00 |
| Silicone Oil | 7.50 |
| Titanium Dioxide | 0.10 |
| Demineralised Water | to 100.00 |

### Example 11

| **Facial Cleanser** | |
|---|---|
| | % **w/w** |
| Potassium dodecanoyl monolactylate | 15.00 |
| Potassium dodecanoyl dilactylate | 15.00 |
| Disodium lauryl sulphosuccinate | 7.00 |
| Glycerol | 5.00 |
| Sodium chloride | 4.20 |
| Methyl gluceth -20 (Humectant/Emollient) | 3.00 |
| Potassium Hexanoyl Dilactylate | 1.00 |
| Polyquaternium -10 (Foam Modifier) | 0.40 |
| Ethyleneglycol monostearate | 0.40 |
| Fragrance | 0.30 |
| Citric acid | to pH 7.0 |
| Distilled water | to 100.00 |

### Example 12

| **Shampoo** | |
|---|---|
| | **% w/w** |
| Triethanolammonium lauryl sarcosinate | 15.00 |
| Sodium lauryl (PEG)-10 acetate | 4.00 |
| Propylene glycol | 2.50 |
| Triethanolammonium heptanoyl monolactylate | 0.50 |
| Citric acid | to pH 6.0 |
| Demineralised water | to 100.00 |

### Example 13

| **Sunscreen Cream** | |
|---|---|
| | **% w/w** |
| Hexanoyl lactylic acid | 1.20 |
| Mineral oil | 20.00 |
| Microcrystalline wax | 5.00 |
| Sorbitan sesquinoleate | 1.50 |
| Oxybentone | 1.00 |
| Parsol 1789 (Sunscreen) | 0.40 |
| Parsol MCX (Sunscreen) | 2.00 |
| Perfume | qs |
| Colour | qs |
| Sodium hydroxide | to pH 4.0 |
| Water | to 100.00 |

### Example 14

| **Sunscreen Cream** | |
|---|---|
| | **% w/w** |
| Zinc hexanoyl monolactylate | 0.75 |
| Polyoxyethylene-2-stearyl alcohol | 4.80 |
| Polyoxyethylene-20-stearyl alcohol | 1.20 |
| Polyoxypropylene-15-stearyl alcohol | 12.00 |
| Parsol MCX (sunscreen) | 6.00 |
| Parsol 1789 (sunscreen) | 3.00 |
| Octyl salicylate | 2.00 |
| Colour | qs |
| Water | to 100.00 |

## Claims

1. The use of an acyl lactylate of the following structure (I) 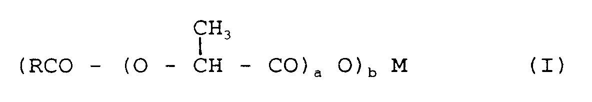 where RCO represents a C₄-C₇ acyl radical; M represents H or a counterion chosen from alkali metal, alkaline earth metal, zinc, silver, ammonium or substituted ammonium having one or more C₁ to C₃ alkyl or hydroxy alkyl group(s); a is an integer of from 1 to 3; b is 1 or 2; or mixtures of such acyl lactylates;
in the preparation of an antimicrobially active composition.

2. The use according to claim 1, in which the composition is a composition intended for topical application to the human body.

3. The use according to claim 2, in which the acyl lactylate is incorporated in the composition in an amount of from 0.01 to 10% by weight, based on the weight of the composition.

4. The use according to claim 1 in which in formula (I) M represents hydrogen, sodium, potassium or calcium.

5. The use according to any one of claims 1 to 4, in which the acyl group in formula (I) is a C₆ acyl group.

## Patentansprüche

1. Verwendung eines Acyllactylats der nachstehenden Struktur (I) 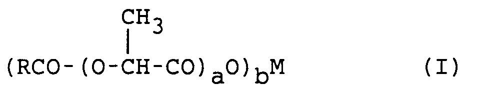 worin RCO einen C₄-C₇-Acylrest wiedergibt; M H oder ein Gegenion, ausgewählt aus Alkalimetall, Erdalkalimetall, Zink, Silber, Ammonium oder mit einer oder mehreren C₁ bis C₃-Alkyl- oder Hydroxyalkylgruppe(n) substituiertes Ammonium wiedergibt; a eine ganze Zahl von 1 bis 3 ist; b 1 oder 2 ist; oder Gemischen solcher Acyllactylate;
bei der Herstellung einer antimikrobiellen Wirkstoffzusammensetzung.

2. Verwendung nach Anspruch 1, wobei die Zusammensetzung zur örtlichen Anwendung auf den menschlichen Körper vorgesehen ist.

3. Verwendung nach Anspruch 2, wobei das Acyllactylat der Zusammensetzung in einer Menge von 0,01 bis 10 Gewichtsprozent, bezogen auf das Gewicht der Zusammensetzung, zugesetzt wird.

4. Verwendung nach Anspruch 1, wobei in Formel (I) M Wasserstoff, Natrium, Kalium oder Calcium wiedergibt.

5. Verwendung nach einem der Ansprüche 1 bis 4, wobei die Acylgruppe in Formel (I) eine C₆-Acylgruppe darstellt.

## Revendications

1. L'utilisation d'un lactylate d'acyle présentant la structure suivante (I) : (I) 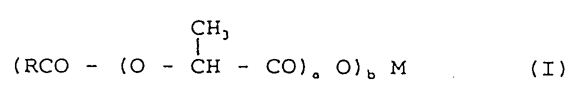 où RCO représente un radical d'acyle en C₄-C₇ ; M représente H ou un contre-ion choisi parmi un métal alcalin, un métal alcalino-terreux, du zinc, de l'argent, de l'ammonium ou de l'ammonium substitué présentant un ou plusieurs groupe(s) alkyle ou hydroxyalkyle en C₁ à C₃ ; a est un nombre entier compris entre 1 et 3 ; b est égal à 1 ou 2 ; ou des mélanges de tels lactylates d'acyle ;
dans la préparation d'une composition d'agents antimicrobes.

2. L'utilisation selon la Revendication 1, dans laquelle la composition est une composition destinée à des applications locales sur le corps humain.

3. L'utilisation selon la Revendication 2, dans laquelle le lactylate d'acyle est incorporé dans la composition dans une proportion comprise entre 0,01 et 10 % en masse, sur la base de la masse de la composition.

4. L'utilisation, en accord avec la Revendication 1, pour laquelle, dans la formule (I), M représente de l'hydrogène, du sodium, du potassium ou du calcium.

5. L'utilisation en accord avec l'une quelconque des Revendications 1 à 4, dans laquelle le groupe acyle dans la formule (I) est un groupe acyle en C₆.
